(19) Europäisches Patentamt
European Patent Office
Office européen des brevets

(11) **EP 2 334 202 B1**

(12) **EUROPEAN PATENT SPECIFICATION**

(45) Date of publication and mention
of the grant of the patent:
**01.02.2012 Bulletin 2012/05**

(21) Application number: **09811073.7**

(22) Date of filing: **28.07.2009**

(51) Int Cl.:
***A23L 1/236*** *(2006.01)*        ***A61K 9/20*** *(2006.01)*

(86) International application number:
**PCT/EP2009/005460**

(87) International publication number:
**WO 2010/025796 (11.03.2010 Gazette 2010/10)**

(54) **TABLETTING OF ERVTHRITOL**

ERYTHRITOL-TABLETTIERUNG

COMPRESSION DE L'ÉRYTHRITOL

(84) Designated Contracting States:
**AT BE BG CH CY CZ DE DK EE ES FI FR GB GR
HR HU IE IS IT LI LT LU LV MC MK MT NL NO PL
PT RO SE SI SK SM TR**

(30) Priority: **04.09.2008 EP 08015598**

(43) Date of publication of application:
**22.06.2011 Bulletin 2011/25**

(73) Proprietor: **Cargill, Incorporated
Wayzata, MN 55391 (US)**

(72) Inventors:
• **BOGHMANS, Catherine, Patricia, L.
B-1852 Beigem (BE)**

• **MEEUS, Liesbeth, Maria, Fernande
B-3078 Everberg (BE)**

(74) Representative: **Elseviers, Myriam
Cargill Europe BVBA
Bedrijvenlaan 9
B-2800 Mechelen (BE)**

(56) References cited:
**EP-A- 0 497 439    EP-A- 0 913 148
EP-A- 0 922 464**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

Technical field

The present invention relates to the preparation of an erythritol containing composition suitable for tabletting.

Background of the invention

With the present interest in the use of sugar-free and/or low calorie products, tablets for pharmaceutical, confectionery or food applications are mostly made with sugar alcohols, such as xylitol, maltitol, sorbitol, mannitol and erythritol.

The tablet does not only contain the drug or a reagent, it also contains other ingredients which act as fillers, such as lactose or phosphates; lubricating agents, such as talc, stearic acid or paraffin and disintegrating agents, such as carboxymethyl-cellulose or starch. For confectionery purposes the tablets often include aroma's and colorants at low concentration.

Direct compression of spray-dried erythritol has been described in European patent EP 0 497 439. The tablets are always prepared with maltodextrin as binder.

European patent application EP 0 528 604 discloses the co-crystallized sorbitol and xylitol and tablets made therefrom.

EP 0 896 528 relates to a polyol composition with high concentration of a non-hygroscopic polyol obtained by spray-drying or fluidized bed granulation.

EP 0 922 464 relates to a process for preparing quickly disintegradable compression-molded materials based upon erythritol. A tablet is obtained by direct compression molding. The thus obtained quickly disintegradable compression molded material is endowed with excellent disintegration and dissolution properties when put in the oral cavity or water.

EP 0 913 148 relates to a process for preparing an erythritol containing composition suitable for use as an excipient for tabletting. The suitable composition was prepared by co-crystallisation of erythritol and a second polyol such as sorbitol. The erythritol was used as such and mixed with sorbitol before co-crystallisation. After the co-crystallisation, the product was milled and tabletted. The process does not involve a granulation step.

There is a further interest for using erythritol with increased specific surface area as an excipient in tablets.

Summary of the invention

The current invention relates to a compressible composition consisting of erythritol having a specific surface area greater than 0.25 m$^2$/g, preferably greater than 0.3 m$^2$/g, more preferably greater than 0.4 m$^2$/g and a binder selected from the group consisting of pregelatinised starch, microcrystalline cellulose, carboxymethyl cellulose, maltose, sorbitol, maltitol, xylitol, isomalt, and mixtures thereof and characterized in that the composition is granulated.

It further relates to a chewable tablet comprising the previously described compressible composition.

Furthermore it relates to a process for preparing the compressible composition of the current invention and it is comprising the following steps:

a) taking erythritol having a specific surface area greater than 0.25 m$^2$/g, preferably greater than 0.3 m$^2$/g, more preferably greater than 0.4 m$^2$/g,
b) adding the binder selected from the group consisting of pregelatinised starch, microcrystalline cellulose, carboxymethyl cellulose, maltose, sorbitol, maltitol, xylitol, isomalt, and mixtures thereof,
c) granulating,
d) optionally wet sieving of the granulated product,
e) drying the granulated product,
f) optionally sieving of the granulated product.

It further describes a process for preparing the tablet according to the current invention and it comprises the following steps:

a) Taking the granulated product prepared according to the current invention
b) Blending with a lubricant,
c) Tabletting at compressing forces from 5 to 30 kN.

Finally it relates to the use of tablet in food, feed, pharma and cosmetic applications.

Detailed description of the invention

The current invention relates to a compressible composition consisting of erythritol having a specific surface area greater than 0.25 $m^2/g$, preferably greater than 0.3 $m^2/g$, more preferably greater than 0.4 $m^2/g$ and a binder selected from the group consisting of pregelatinised starch, microcrystalline cellulose, carboxymethyl cellulose, maltose, sorbitol, maltitol, xylitol, isomalt, and mixtures thereof and characterized in that the composition is granulated.

Erythritol is a tetriitol which is obtainable via chemical processes, preferably other than hydrogenation of carbohydrates, and/or microbial processes or fermentation, preferably fermentation. Any erythritol is suitable provided that its specific surface area is greater than 0.25 $m^2/g$. Without any limitation, a suitable source of erythritol having a specific surface area greater than 0.25 $m^2/g$, is a micronized erythritol prepared as described in WO2009016133, or turbomilled erythritol and the like, or a fine grade of erythritol provided that its specific surface area is greater than 0.25 $m^2/g$.

The specific surface area is measured with BET method.

Surprisingly it was found that the specific surface area has a positive effect on the subsequent granulation, even with a binder in liquid form. The bigger the specific surface area the better the granulation is performed. Granulation is a process in which primary powder particles are made to form larger entities called granules. The granulation allows preventing segregation of the constituents of the powder mix, to improve the flow properties of the powder mix, and to improve the compaction characteristics of the powder mix.

Granulation methods can be divided in two basic types, namely wet methods, which use a liquid in the process, and dry methods in which no liquid is used. Wet granulation is most often used and involves many steps, including: agglomerating (granulating) of dry primary powder particles of active ingredients and excipients in the presence of a granulating fluid upon agitation using low-shear or high-shear mixers or fluidized beds, wet sieving (wet screening) to remove larger lumps, drying the granulated product, and milling or sieving (screening) the dried granulated product to achieve a granulated product having the desired granule size distribution. The obtained granulation may subsequently be tabletted.

Furthermore the erythritol is having a volume mean diameter, reference to Ph.Eur.VI, of less than 100 $\mu$m, preferably less than 50 $\mu$m, more preferably less than 40 $\mu$m. The binders such as pregelatinised starch, microcrystalline cellulose, carboxymethyl cellulose, maltose or mixtures thereof are added in dry form, whereas the binder such as sorbitol is added in liquid form. Binders such as maltitol, isomalt, xylitol, or mixtures thereof, can be added in dry or liquid form. The preferred binder is liquid sorbitol in a dry matter concentration of at least 50%, preferably 60%, more preferably at least 70%.

The ratio of erythritol to binder is 50 % to 50%, preferably 70% to 30% up to 90 % to 10 % dry weight. Preferably, with liquid sorbitol as binder, the ratio of erythritol and sorbitol is from 50 % to 50%, preferably 70% to 30% up to 90 % to 10 % dry weight.

The composition is further characterized in that it has a moisture pick-up below 3%, preferably below 2.7% at 65% relative humidity, at 25°C.

Furthermore, the current invention relates to the use in food applications, feed, pharma applications, cosmetics, detergents, fertilizer or agrochemical products. In fact, without being limiting, the compressible composition of the current invention can be used in food products, animal feed, health food, dietetic products, animal medicine, with bath agent, in agrochemical products, with fertilizer, with plant granules, with plant seeds or seed grains, and any other product being it ingested by humans and/or animals or any other product which can benefit from the improved properties of the compressible composition of the current invention. The compressible composition of the current invention can be used as carrier for additives based on enzymes or microorganisms, detergent tablets, vitamins, flavors, perfumes, acids, sweeteners or various active ingredients with medicinal or non-medicinal applications. Eventually mixtures of additives can be applied.

It further relates to a chewable tablet comprising the previously described compressible composition. The term "tablet", as used herein, includes any tablet, in particular tablets in any form, shape and of any physical, chemical or sensory property, and tablets for any route of administration, indication and application. The tablets produced according to the invention is a chewable tablet. A chewable tablet according to the present invention is a soft tablet where chewing helps to break the tablet particles and release the active ingredient, flavor, aroma or the like, in the mouth before swallowing. A chewable tablet dosage form can be a soft pill, tablet, gum and more recently "chewy squares". The tablet hardness and friability are highly important properties of a chewable tablet comprising active ingredient(s) and having desirable chewability properties.

Said tablets can be applied in food, feed, cosmetics, detergents and/or pharma applications. The chewable tablet is significant different from a quickly disintegradable tablet in the oral cavity or in water and has a different purpose to serve.

As a lubricant agent in tablet formation, magnesium stearate, calcium stearate, stearic acid, sucrose fatty acid esters, and/or talc and the like can be added according to needs. Furthermore surface active agents such as sodium lauryl sulfate, propylene glycol, sodium dodecanesulfonate, sodium oleate sulfonate, and sodium laurate mixed with stearates and talc, sodium stearyl fumarate, sucrose fatty acid esters, and the like can be added according to needs.

The thus obtained tablets have a friability of 0.3 to 0.7 %, at a compression force from 5 to 30kN, preferably from 0.3 to 0.5 %, according to Ph. Eur. VI. Preferably these tablets have a surface of at least 1 cm$^2$ and a weight of 350 mg, a surface of up to 2 cm$^2$ and a weight of 1090 mg.

The tensile strength of these tablets can be expressed in function of compression force. A tensile strength between 1.5 and 3.10 N/mm$^2$, even up to 3.60 N/mm$^2$ is obtainable at a compression force of 5 to 30 kN. The tensile strength at 20 kN is at least 2.5 N/mm$^2$, preferably at least 2.8 N/mm$^2$, more preferably at least 2.9 N/mm$^2$, at least 3.0 N/mm$^2$, at least 3.1 N/mm$^2$, at least 3.2 N/mm$^2$, most preferably at least 3.5 N/mm$^2$ .The tablets have a hardness of at least 110 N, preferably at least 130 N, more preferably at least 145 N at a compression force of 15 kN. Preferably the tablets have a surface of at least 1 cm$^2$ and a weight of 350 mg, a surface of up to 2 cm$^2$ and a weight of 1090 mg.

The chewable tablets of the current invention have a friability of 0.3 to 0.7 %, at a compression force from 5 to 30kN, and a tensile strength of between 1.5 and 3.10 N/mm$^2$, even up to 3.60 N/mm$^2$ is obtainable at a compression force of 5 to 30 kN.

Furthermore it relates to a process for preparing the compressible composition of the current invention and it is comprising the following steps:

a) taking erythritol having a specific surface area greater than 0.25 m$^2$/g, preferably greater than 0.3 m$^2$/g, more preferably greater than 0.4 m$^2$/g,
b) adding the binder selected from the group consisting of pregelatinised starch, microcrystalline cellulose, carboxymethyl cellulose, maltose, sorbitol, maltitol, xylitol, isomalt, and mixtures thereof,
c) granulating,
d) optionally wet sieving of granulated product,
e) drying the granulated product,
f) optionally sieving of the granulated product.

In the process, preferably erythritol is having further a volume mean diameter of less than 100 μm, preferably less than 50 μm, more preferably less than 40 μm. When starting with more coarse material, a milling step, preferably turbomilling, is included in the process in order to obtain a volume mean diameter of less than 100 μm, preferably less than 50 μm, more preferably less than 40 μm. The thus obtained product has a specific surface area greater than 0.25 m$^2$/g, preferably greater than 0.3 m$^2$/g, more preferably greater than 0.4 m$^2$/g, and it turns out to have a positive effect on the subsequent granulation.

The binders such as pregelatinised starch, microcrystalline cellulose, carboxymethyl cellulose, maltose or mixtures thereof are added in dry form, whereas the binder such as sorbitol is added in liquid form. Binders such as maltitol, isomalt, xylitol or mixtures thereof, can be added in dry or liquid form. Preferably the binder is liquid sorbitol and it is added at a dry substance concentration of at least 50%, preferably at least 60%, more preferably at least 70%. Depending upon the volume mean diameter and the moisture content of the blend, the product is sieved and/or dried.

The granules formed in step c) of the current process are optionally pressed through a sieve of a predetermined size. Preferably a screening machine is applied for this sieving. At the same time or thereafter the product is dried.

Any drier type can be applied for drying of the granules, but preferably a fluid bed is applied for this purpose. The sufficiently dry product is granulated in a typical granulator.

It further describes a process for preparing the tablet according to the current invention and it comprises the following steps:

a) Taking the granulated product prepared according to the current invention,
b) Blending with a lubricant,
c) Tabletting at compressing forces from 5 to 30 kN.

The granulated product (= compressible composition) is further blended with a suitable lubricant, preferably magnesium stearate and tabletted in a tabletting machine.

Finally it relates to the use of tablet in food, feed, pharma and cosmetic applications.

If tablets are prepared for food (confectionery) applications than in general up to about 99 % (w/w) consists of the erythritol containing compressible composition and aroma, colourant, flavour and a lubricating agent, are added. If tablets are prepared for pharmaceutical applications an active ingredient such as a drug is added and fillers, lubricating agents or disintegrating agents are added if needed.

The invention will hereunder be illustrated in the form of a series of non-limiting examples.

Examples

Methods for evaluating granule and tablet properties

The granules were characterized by their volume mean diameter (size distribution). The following measurement method was employed.

Size distribution. Size distribution was determined according to the European Pharmacopoeia VI Test method 2.9.31 using a laser light particle sizer, type Helos KF - Rodos T4.1, of Sympatec GmbH (Germany). The particle size was analysed by laser light diffraction.

The tablets were characterized by their hardness and friability. For each compression force, 10 tablets for hardness and 19 tablets for friability were analyzed and mean values were calculated. The following measuring methods were employed.

Hardness. Hardness, i.e. the diametral crushing strength, was determined according to the European Pharmacopoeia VI Test method 2.9.8 Resistance to crushing of tablets by using a conventional pharmaceutical hardness tester (hardness tester model Multicheck V, available from Erweka GmbH (Germany)). In order to compare values across different size tablets, the breaking strength was normalized for the area of the break. The normalized value, expressed as $N/mm^2$, is herein referred to as tensile strength (Ts) and calculated as follows:

$$Ts = 2H/\pi TD.$$

wherein H is the hardness, T the thickness and D the diameter of the tablet. For each compression force, 10 tablets were analyzed on hardness (H), thickness (T) and diameter (D).

Friability. Friability measurements were determined according to the European Pharmacopoeia VI Test method 2.9.7 Friability of uncoated tablets.

Example 1A

Coarse erythritol product (Cargill C*PharmEridex 16956) was milled in a Bauermeister turbo mill UTL at a 1 mm sieve and powder with a volume mean diameter of 30 $\mu$m was obtained. The volume mean diameter was determined with laser diffraction. The erythritol had a specific surface area of 0.40 $m^2/g$.

500 g of the milled erythritol powder was dry blended in a high Shear Mixer (Pro-C-ept - Mi-Pro, Chopper: 3000 rpm and Impeller: 1200 rpm) for 60 seconds.

79.17 g of liquid sorbitol (at 70% dry substance) (Cargill C*PharmSorbidex NC 16205) was added in droplets at 9.5 g/min). After the addition of the liquid sorbitol, the mixing of the blend was continued for 60 seconds.

The granulated powder was manually wet screened over a 2 mm sieve.

The wet sieved granules were dried in the fluid bed (Aeromatic-Fielder GEA - Strea-1) for 30 minutes at a temperature of 70°C.

The dried granules were screened in the granulator (Erweka (FGS + AR400E) over a sieve of 0.500 mm for 5 to 10 minutes at 100 turns per minute

The dry sieved granules were then blended with 3% of magnesium stearate in a Pharmatech Equipment at 28 rpm.

Example 1B

Coarse erythritol product (Cargill ZeroseTM 16952) was milled in a Bauermeister turbo mill UTL at a 1 mm sieve and powder with a volume mean diameter of 20 $\mu$m was obtained. The volume mean diameter was determined with laser diffraction. The erythritol had a specific surface area of 0.45 $m^2/g$.

500 g of the milled erythritol powder was dry blended in a high Shear Mixer (Pro-C-ept - Mi-Pro, Chopper: 3000 rpm and Impeller: 1200 rpm) for 60 seconds.

79.17 g of liquid sorbitol (at 70% dry substance) (Cargill C*PharmSorbidex NC 16205) was added in droplets at 9.5 g/min). After the addition of the liquid sorbitol, the mixing of the blend was continued for 60 seconds.

The granulated powder was manually wet screened over a 2 mm sieve.

The wet sieved granules were dried in the fluid bed (Aeromatic-Fielder GEA - Strea-1) for 30 minutes at a temperature of 70°C.

The dried granules were screened in the granulator (Erweka (FGS + AR400E) over a sieve of 0.500 mm for 5 to 10 minutes at 100 turns per minute

The dry sieved granules were then blended with 3% of magnesium stearate in a Pharmatech Equipment at 28 rpm.

Example 2A

The granulated product obtained in example 1A was then tabletted in a tabletting machine (Korsch - PH100) at compression forces varying from 5 kN to 30 kN.
Tablets had a surface of 1 cm$^2$, the diameter of the tablet was 11.3 mm and the weight is 350 mg.
The thus obtained tablets were analysed as follows:

Friability comparison

| Compression Force (kN) | Product from example 2A % | Comparison with product from example 1 in EP0913148 % |
|---|---|---|
| 5 | 0.49 | 1.85 |
| 10 | 0.38 | 1.05 |
| 15 | 0.52 | 0.91 |
| 20 | 0.44 | 0.96 |
| 25 | 0.63 | 0.86 |
| 30 | 0.55 | 0.94 |

Tensile strength comparison

| Compression Force (kN) | Product from example 2A (N/mm$^2$) | Comparison with product from example 1 in EP0913148 (N/mm$^2$) |
|---|---|---|
| 5 | 1.55 | 0.99 |
| 10 | 2.35 | 1.99 |
| 15 | 2.87 | 2.46 |
| 20 | 2.69 | 2.66 |
| 25 | 2.47 | 2.62 |
| 30 | 2.45 | 2.53 |

Hardness comparison

| Compression Force (kN) | Product from example 2A (N) | Comparison with product from example 1 in EP0913148 (N) |
|---|---|---|
| 5 | 78 | 50 |
| 10 | 113 | 94 |
| 15 | 133 | 114 |
| 20 | 133 | 122 |
| 25 | 118 | 121 |
| 30 | 117 | 117 |

Example 2B

The granulated product obtained in example 1 B was then tabletted in a tabletting machine (Korsch - PH100) at compression forces varying from 5 kN to 30 kN.
Tablets had a surface of 1 $cm^2$, the diameter of the tablet was 11.3 mm and the weight is 350 mg.
The thus obtained tablets were analysed as follows:

Friability comparison

| Compression Force (kN) | Product from Example 2B (%) | Comparison with product from example 1 in EP0913148 (%) |
|---|---|---|
| 5 | 0.56 | 1.85 |
| 15 | 0.34 | 0.91 |
| 20 | 0.27 | 0.96 |
| 25 | 0.39 | 0.86 |
| 30 | 0.38 | 0.94 |

Tensile strength comparison

| Compression Force (kN) | Product from Example 2B (N/mm23) | Comparison with product from example 1 in EP0913148 (N/mm$^2$) |
|---|---|---|
| 5 | 1.00 | 0.99 |
| 10 | 2.30 | 1.99 |
| 15 | 3.03 | 2.46 |
| 20 | 3.31 | 2.66 |
| 25 | 3.64 | 2.62 |
| 30 | 3.53 | 2.53 |

Hardness comparison

| Compression Force (kN) | Product from Example 2B (N) | Comparison with product from example 1 in EP0913148 (N) |
|---|---|---|
| 5 | 51 | 50 |
| 10 | 111 | 94 |
| 15 | 143 | 114 |
| 20 | 155 | 122 |
| 25 | 169 | 121 |
| 30 | 165 | 117 |

Example 3A

Coarse erythritol product (Cargill C*PharmEridex 16956) was milled in a Bauermeister turbo mill UTL at a 1 mm sieve and powder with a volume mean diameter of 30 μm was obtained. 250 g of the milled erythritol powder was dry blended with 250 g paracetamol (fines) in a high Shear Mixer (Pro-C-ept - Mi-Pro, Chopper: 3000 rpm and Impeller: 1200 rpm) for 60 seconds.

74.42 g of liquid sorbitol (at 70% dry substance) (Cargill C*PharmSorbidex NC 16205) was added in droplets at 9.5 g/min). After the addition of the liquid sorbitol, the mixing of the blend was continued for 60 seconds.

The granulated powder was manually wet screened over a 2 mm sieve.

The wet sieved granules were dried in the fluid bed (Aeromatic-Fielder GEA - Strea-1) for $3_0$ minutes at a temperature of 70°C.

The dried granules were screened in the granulator (Erweka (FGS + AR400E) over a sieve of 0.500 mm for 5 to 10 minutes at 100 turns per minute

The dry sieved granules were then blended with 3% of magnesium stearate in a Pharmatech Equipment at 28 rpm.

Example 3B

Coarse erythritol product (Cargill ZeroseTM 16952) was milled in a Bauermeister turbo mill UTL at a 1 mm sieve and powder with a volume mean diameter of 20 μm was obtained.

250 g of the milled erythritol powder was dry blended with 250 g paracetamol (fines) in a high Shear Mixer (Pro-C-ept - Mi-Pro, Chopper: 3000 rpm and Impeller: 1200 rpm) for 60 seconds.

74.42 g of liquid sorbitol (at 70% dry substance) (Cargill C*PharmSorbidex NC 16205) was added in droplets at 9.5 g/min). After the addition of the liquid sorbitol, the mixing of the blend was continued for 60 seconds.

The granulated powder was manually wet screened over a 2 mm sieve.

The wet sieved granules were dried in the fluid bed (Aeromatic-Fielder GEA - Strea-1) for 30 minutes at a temperature of 70°C.

The dried granules were screened in the granulator (Erweka (FGS + AR400E) over a sieve of 0.500 mm for 5 to 10 minutes at 100 turns per minute

The dry sieved granules were then blended with 3% of magnesium stearate in a Pharmatech Equipment at 28 rpm.

Example 4A

The granulated product obtained in example 3A was then tabletted in a tabletting machine (Korsch - PH100) at compression forces varying from 10 kN to 30 kN, and gave a tensile strength of 1.8N/mm$^2$ at 20 kN compression force. Tablets had a surface of 2 cm$^2$, the diameter of the tablet was 16 mm and the weight is 1090 mg.

Example 4B

The granulated product obtained in example 3B was then tabletted in a tabletting machine (Korsch - PH100) at compression forces varying from 10 kN to 30 kN, and gave a tensile strength of 2.7 N/mm$^2$ at 20 kN compression force. Tablets had a surface of 1 cm$^2$, the diameter of the tablet was 11.3 mm and the weight is 350 mg.

**Claims**

1. Compressible composition consisting of erythritol having a specific surface area greater than 0.25 m$^2$/g, preferably greater than 0.3 m$^2$/g, more preferably greater than 0.4 m$^2$/g and a binder selected from the group consisting of pregelatinised starch, microcrystalline cellulose, carboxymethyl cellulose, maltose, sorbitol, maltitol, xylitol, isomalt, and mixtures thereof, **characterized in that** the composition is granulated.

2. A composition according to claim 1 **characterized in that** erythritol is having a volume mean diameter of less than 100 μm, preferably less than 50 μm, more preferably less than 40 μm.

3. A composition according to claim 1 or 2 **characterized in that** erythritol and sorbitol are present in a ratio of 50 % to 50% to 90 % to 10 % dry weight.

4. A composition according to anyone of claims 1 to 3 **characterized in that** it has a moisture pick-up below 3%, preferably below 2.7% at 65% relative humidity, at 25°C.

5. A chewable tablet comprising a composition according to anyone of claims 1 to 4.

**6.** A tablet according to claim 5 **characterized in that** it has a friability of 0.3 to 0.7 %, at a compression force from 5 to 30kN, preferably from 0.3 to 0.5 %.

**7.** A tablet according to claim 6 **characterized in that** it has a tensile strength at 20 kN of at least 2.5 N/mm$^2$, preferably at least 2.8 N/mm$^2$, more preferably at least 3.3 N/mm$^2$.

**8.** A process for preparing a compressible composition according to anyone of claim 1 to 4:

a) taking erythritol having a specific surface area greater than 0.25 m$^2$/g, preferably greater than 0.3 m$^2$/g, more preferably greater than 0.4m$^2$/g,
b) adding the binder selected from the group consisting of pregelatinised starch, microcrystalline cellulose, carboxymethyl cellulose, maltose, sorbitol, maltitol, xylitol, isomalt, and mixtures thereof,
c) granulating,
d) optionally wet sieving of granulated product,
e) drying the granulated product,
f) optionally sieving of the granulated product.

**9.** A process for preparing a tablet according to anyone of claim 5 to 7:

a) Taking the granulated product of process according to claim 8,
b) Blending with a lubricant,
c) Tabletting at compressing forces from 5 to 30 kN.

**10.** A process according to claim 9 **characterised in that** an active ingredient is added in step a) and/or b).

**11.** Use of chewable tablet according to anyone of claims 5 to 7 in food, feed, pharma and cosmetic applications.


**Patentansprüche**

**1.** Komprimierbare Zusammensetzung, bestehend aus Erythritol mit einer spezifischen Oberfläche von mehr als 0,25 m$^2$/g, vorzugsweise mehr als 0,3 m$^2$/g, insbesondere mehr als 0,4 m$^2$/g und einem Bindemittel, ausgewählt aus vorgelantinierter Stärke, mikrokristalliner Cellulose, Carboxymethylcellulose, Maltose, Sorbitol, Maltitol, Xylitol, Isomalt und Mischungen daraus, **dadurch gekennzeichnet, dass** die Zusammensetzung granuliert ist.

**2.** Zusammensetzung nach Anspruch 1, **dadurch gekennzeichnet, dass** Erythritol einen durchschnittlichen Volumendurchmesser von weniger als 100 μm, vorzugsweise weniger als 50 μm, insbesondere weniger als 40 μm aufweist.

**3.** Zusammensetzung nach Anspruch 1 oder 2, **dadurch gekennzeichnet**, das Erythritol und Sorbitol, in einem Verhältnis von 50 % zu 50 % zu 90 % zu 10 % Trockengewicht vorhanden sind.

**4.** Zusammensetzung nach einem der Ansprüche 1 bis 3, **dadurch gekennzeichnet, dass** sie eine Feuchtigkeitsaufnahme von weniger als 3 %, vorzugsweise weniger als 2,7 % bei 65 % relativer Feuchtigkeit bei 25 °C aufweist.

**5.** Kautablette, umfassend eine Zusammensetzung nach einem der Ansprüche 1 bis 4.

**6.** Tablette nach Anspruch 5, **dadurch gekennzeichnet, dass** sie eine Sprödigkeit von 0,3 bis 0,7 % bei einer Druckstärke von 5 bis 30 kN, vorzugsweise von 0,3 bis 0,5 % aufweist.

**7.** Tablette nach Anspruch 6, **dadurch gekennzeichnet, dass** sie eine Zugfestigkeit bei 20 kN von mindestens 2,5 N/mm$^2$, vorzugsweise mindestens 2,8 N/mm$^2$, insbesondere mindestens 3,3 N/mm$^2$ aufweist.

**8.** Verfahren zur Herstellung einer komprimierbaren Zusammensetzung nach einem der Ansprüche 1 bis 4, durch

a) Verwenden von Erythritol mit einer spezifischen Oberfläche von mehr als 0,25m$^2$/g, vorzugsweise mehr als 0,3 m$^2$/g, insbesondere mehr als 0,4 m$^2$/g,
b) Zugeben des Bindemittels, ausgewählt aus der Gruppe bestehend aus vorgelantinierter Stärke, mikrokristalliner Cellulose, Carboxymethylcellulose, Maltose, Sorbitol, Maltitol, Xylitol, Isomalt und Mischungen daraus,
c) Granulieren,
d) wahlweise Nasssieben des granulierten Produkts,
e) Trocknen des granulierten Produkts,
f) wahlweise Sieben des granulierten Produkts.

**9.** Verfahren zur Herstellung einer Tablette nach einem der Ansprüche 5 bis 7 durch:

a) Verwenden des granulierten Produkts des Verfahrens nach Anspruch 8,
b) Mischen mit einem Schmiermittel,
c) Tablettieren bei Druckkräften von 5 bis 30 kN.

**10.** Verfahren nach Anspruch 9, **dadurch gekennzeichnet, dass** in Schritt a) und/oder b) ein Wirkstoff zugegeben wird.

**11.** Verwendung einer Kautablette nach einem der Ansprüche 5 bis 7 in Nahrungsmittel-, pharmazeutischen und kosmetischen Anwendungen.

## Revendications

**1.** Composition compressible consistant en érythritol ayant une surface spécifique supérieure à 0,25 m$^2$/g, de préférence supérieure à 0,3 m$^2$/g, de manière davantage préférée, supérieure à 0,4 m2lg et un liant choisi dans le groupe comprenant l'amidon prégélatinisé, la cellulose microcristalline, la carboxyméthylcellulose, le maltose, le sorbitol, le maltitol, le xylitol, fisomalt et leurs mélanges, **caractérisée en ce que** la composition est granulée.

**2.** Composition selon la revendication 1, **caractérisée en ce que** l'érythritol a un diamètre volumique moyen inférieur à 100 $\mu$m, de préférence, inférieur à 50 $\mu$m, de manière davantage préférée, inférieur à 40 $\mu$m.

**3.** Composition selon la revendication 1 ou 2, **caractérisée en ce que** l'érythritol et le sorbitol sont présents en un rapport de 50% - 50% à 90% - 10% de poids sec.

**4.** Composition selon l'une quelconque des revendications 1 à 3, **caractérisée en ce qu'**elle a un taux d'absorption d'humidité inférieur à 3%, de préférence inférieur à 2,7% à 65% d'humidité relative, à 25°C.

**5.** Comprimé à mâcher comprenant une composition selon l'une quelconque des revendications 1 à 4.

**6.** Comprimé selon la revendication 5, **caractérisé en ce qu'**il a une friabilité de 0,3 à 0,7% à une force de compression de 5 à 30 kN, de préférence de 0,3 à 0,5%.

**7.** Comprimé selon la revendication 6, **caractérisé en ce qu'**il a une résistance à la traction à 20 kN d'au moins 2,5 N/mm$^2$, de préférence d'au moins 2,8 Nlmm$^2$, de manière davantage préférée, d'au moins 3,3 N/mm$^2$.

**8.** Procédé de préparation d'une composition compressible selon l'une quelconque des revendications 1 à 4 consistant à :

a) se munir d'érythritol ayant une surface spécifique supérieure à 0,25 m$^2$/g, de préférence supérieure à 0,3 m$^2$/g, de manière davantage préférée, supérieure à 0,4 m$^2$/g,
b) ajouter le liant choisi dans le groupe comprenant l'amidon prégélatinisé, la cellulose microcristalline, la carboxyméthylcellulose, le maltose, le sorbitol, le maltitol, le xylitol, l'isomalt et leurs mélanges,
c) granuler,
d) éventuellement, tamiser à l'état humide le produit granulé,
e) sécher le produit granulé,
f) éventuellement, tamiser le produit granulé.

**9.** Procédé de préparation d'un comprimé selon l'une quelconque des revendications 5 à 7 consistant à :

a) se munir du produit granulé selon la revendication 8,
b) mélanger avec un lubrifiant,

c) pastiller à des forces de compression de 5 à 30 kN.

**10.** Procédé selon la revendication 9, **caractérisé en ce qu'**un ingrédient actif est ajouté à l'étape a) et/ou b).

**11.** Utilisation d'un comprimé à mâcher selon l'une quelconque des revendications 5 à 7 dans les applications alimentaires, alimentaires animales, pharmaceutiques et cosmétiques.

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0497439 A
- EP 0528604 A
- EP 0896528 A
- EP 0922464 A
- EP 0913148 A
- WO 2009016133 A